# DEMANDE DE BREVET EUROPEEN

(11) **EP 3 299 004 A1**
(43) Date de publication de la demande: **28.03.2018**
(21) Numéro de dépôt: 17192383.2
(22) Date de dépôt: 21.09.2017
(51) Int. Cl.: A61H 5/00, A61B 3/00, A61B 3/02, A61B 3/117

(54) **APPAREIL DE RÉÉDUCATION NEUROSENSORIELLE**

(30) Priorité: 22.09.2016 FR 1658912
(71) Demandeur: Salomon, Georgette, 34800 Canet (FR); Costa, Pascal, 34120 Castelnau de Guers (FR)
(72) Inventeur: Salomon, Georgette, 34800 Canet (FR); Costa, Pascal, 34120 Castelnau de Guers (FR)
(74) Mandataire: Pontet Allano & Associes

(57) **Abrégé**

La présente invention a pour objet un appareil de rééducation neurosensorielle de type banc d'optique pour le dépistage et la rééducation de troubles neurosensoriels. L'invention se situe donc dans le domaine des dispositifs de rééducation fonctionnelle.

Un appareil de rééducation selon l'invention comprend une poutre horizontale comprenant :
- un dispositif porte-filtre comportant des moyens d'obturation,
- un dispositif porte-tests comprenant des moyens de support de signes et/ou de couleurs,
- un moyen d'éclairage projectif dudit dispositif porte-tests, ledit moyen d'éclairage émettant un flux lumineux dont la température de couleur est comprise entre 4 500 K et 6 500 K
- une enceinte opaque dans laquelle sont intégrés lesdits moyen d'éclairage, dispositif porte-filtre et dispositif porte-tests.

## Description

La présente invention a pour objet un appareil de rééducation neurosensorielle de type banc d'optique, pour le dépistage et la rééducation de troubles neurosensoriels. L'invention se situe donc dans le domaine des dispositifs de rééducation fonctionnelle.

L'évolution de la vie moderne amène des désordres importants liés notamment à l'utilisation d'agents non naturels pour l'humain, comme des rayonnements ionisants et des ondes électromagnétiques, présents dans l'air, l'eau et le sol. Ces pollutions sont dangereuses pour le système nerveux, dont l'organe principal est le cerveau, et notamment pour les centres nerveux situés à la base du cerveau, tels que le mésendiencéphale ou système nerveux central. Cette exposition et surexcitation est susceptible de dérégler nos 3 systèmes de vie :
- sensorielle : l'information est captée sous forme de vibration de fréquence d'onde qui sont transformées en influx nerveux,
- végétative : via le système nerveux autonome, et
- psychique : qui concerne l'aspect intellectuel, la concentration, la mémorisation et la vigilance.

Ces dérèglements participent et s'ajoutent à de nombreux troubles fonctionnels dus aux prédispositions héréditaires, au surmenage, aux chocs émotionnels et/ou physiques tels que notamment:
- Angoisses, stress, phobies, hyperémotivité,
- Troubles du sommeil, insomnie, énurésie,
- Bégaiement, strabisme, dyslexie
- Migraines, troubles hormonaux, anorexie, boulimie,
- Troubles de l'hyperactivité avec déficit de l'attention,
- Troubles obsessionnels compulsifs (TOC)
- Dépression, etc....

Les traitements de ces désordres ou pathologies fonctionnelles par des anxiolytiques, somnifères, antidépresseurs et autres traitements médicamenteux peuvent présenter des effets secondaires non négligeables, notamment des troubles digestifs, des maux de tête, une somnolence et/ou une accoutumance, et montrent dans certains cas une limite d'efficacité.-

On connaît une méthode de dépistage et de rééducation, mise au point par Monsieur Georges Quertant et décrite en 1930, qui établit que les différents troubles fonctionnels précédemment cités ont pour origine un dérèglement du système nerveux central. Cette méthode, intitulée «Neuro-pédagogie culture psycho-sensorielle pour l'étude des troubles visuels et leur normalisation » met en corrélation le bon fonctionnement du système nerveux central avec la bonne coordination de la fonction visuelle.

On connaît également un banc d'optique pour la mise en oeuvre de ladite méthode, décrit dans le brevet français N° 2 674 427. Ledit banc d'optique comprend une poutre, montée sur un pied-support réglable, sur laquelle sont disposés successivement les accessoires suivants : un appui frontal et une mentonnière, une barrette escamotable destinée à supprimer certains axes lumineux, une plaque porte-filtre, des moyens d'éclairage et une plaque porte-tests.

Ce banc d'optique est utilisé, d'une part, pour dépister les éventuels troubles du système nerveux central à partir du contrôle des erreurs de la fonction visuelle et, d'autre part, pour la rééducation de cette fonction. Le banc d'optique est en effet utilisé dans des exercices destinés à rétablir une fonction visuelle normale, dans lesquels le patient s'entraîne à percevoir correctement des images tests de difficulté croissante, pendant une durée déterminée pouvant atteindre 30 minutes.

Le brevet français N° 2 674 427 ne divulgue pas la nature des moyens d'éclairages mis en oeuvre dans ledit banc d'optique. Les bancs d'optique utilisés pour le dépistage et la rééducation selon cette méthode de rééducation mettent en oeuvre un éclairage de type « lumière jaune » ou « lumière artificielle ». Ce type d'éclairage peut générer une certaine fatigue visuelle, éventuellement cumulée avec la fatigue visuelle du patient liée à la visualisation sur écran pendant une longue durée dans sa vie quotidienne actuelle.

Les Inventeurs ont maintenant apporté différents perfectionnements inattendus audit banc d'optique, pour développer un appareil de dépistage et de rééducation neurosensorielle intégrant différentes approches thérapeutiques. L'utilisation d'un appareil selon l'invention combine les apports de la rééducation visuelle telle que décrite ci-dessus et ceux de la luminothérapie, auxquels peut s'ajouter une stimulation auditive ou musicothérapie. De plus, l'utilisation d'un appareil selon l'invention pour appliquer ces différentes thérapeutiques intègre les principes de la médecine traditionnelle chinoise. Une telle synergie thérapeutique optimise la rééducation du système nerveux central.

La gamme de troubles fonctionnels pouvant être ainsi traitée peut ainsi être élargie. Ces perfectionnements peuvent également permettre une durée plus courte de rééducation.
Les perfectionnements techniques apportés présentent en outre l'avantage de faciliter la mise en oeuvre du dépistage et de la rééducation, tout en améliorant grandement le confort du patient et du thérapeute.

Par rapport au banc d'optique déjà connu dans l'état de la technique, les perfectionnements apportés sont principalement les suivants :
- un moyen d'éclairage du dispositif porte-tests reproduisant la lumière du jour,
- la présence d'une enceinte opaque intégrant le moyen d'éclairage ainsi que les dispositifs porte-filtre et porte-tests.

Selon différents aspects particuliers de l'invention, d'autres perfectionnements ont également été apportés, et peuvent être apportés seuls ou combinés dans un appareil selon l'invention :
- un moyen de type « pied-support » permettant un réglage en hauteur de grande amplitude de la poutre horizontale du banc d'optique,
- la présence d'au moins un manche ou une poignée, destinée à la stabilisation du patient,
- un appui nez réglable en hauteur,
- un dispositif escamotable comprenant un objet réflecteur ou un éclairage, de couleur rouge, destiné à permettre une correction automatique de la vision,
- une prise pour casque audio/stéréo,
- une minuterie destinée à contrôler précisément la durée de l'exercice,
- un potentiomètre d'intensité, permettant d'adapter l'intensité lumineuse appliquée lors des exercices,
- une ou deux lames escamotables, ladite ou lesdites lame(s) étant rectilignes ou éventuellement munies d'encoches latérales, et
- un dispositif porte-pierres, pour intégrer des pierres naturelles translucides dans le champ de vision du patient.

### DESCRIPTION DETAILLEE

Les figures 1 à 5 jointes sont données à titre d'exemples indicatifs et non limitatifs. Elles représentent chacune un mode de réalisation préféré d'un appareil selon l'invention.
La figure 1 montre une représentation schématique d'un appareil de rééducation neurosensorielle selon l'invention, selon l'exemple 1.
La figure 2 montre un schéma « par transparence » d'un appareil de rééducation neurosensorielle selon l'invention, selon l'exemple 1.
La figure 3 montre une représentation schématique d'un dispositif escamotable comprenant un objet réflecteur ou un éclairage de couleur rouge.
La figure 4 montre une représentation schématique d'un appareil de rééducation neurosensorielle selon l'exemple 2.
La figure 5 montre une représentation schématique de l'arrière d'un dispositif porte-filtre (5), selon l'exemple 2.

La présente invention a pour objet un appareil de rééducation de type banc d'optique comprenant une poutre horizontale (1) comprenant :
- un dispositif porte-filtre (5) comportant des moyens d'obturation (5A, 5B),
- un dispositif porte-tests (7), comprenant des moyens de support de signes et/ou de couleurs,
- un moyen d'éclairage projectif (6) dudit dispositif porte-tests (7),
- une enceinte opaque (4),
ledit appareil de rééducation étant caractérisé en ce que :
- ledit moyen d'éclairage (6) émet un flux lumineux dont la température de couleur est comprise entre 4 500 K et 6 500 K et
- ledit moyen d'éclairage (6), le dispositif porte-filtre (5) et le dispositif porte-tests (7) sont intégrés dans ladite enceinte opaque (4).

Par « moyen d'éclairage projectif du dispositif porte-test » on entend un moyen destiné à projeter un éclairage sur ledit dispositif porte test (7). De préférence, ledit moyen d'éclairage projectif est situé hors de l'axe dudit banc d'optique.

Le degré de blancheur d'un flux lumineux est caractérisé par sa « température » ou « température de couleur » et représenté en unités kelvin (K). La couleur de la lumière du jour varie entre 4 000 et 7 500 K, notamment selon les conditions météorologiques.

La lumière artificielle des ampoules traditionnelles ne correspond qu'à une infime partie du spectre lumineux, et la lumière dite artificielle est caractérisée par une température de 3 000 K à 4 000 K environ. Au contraire, la lumière émise par une lampe de type « lumière du jour », également désignée par le terme « ampoule plein spectre » reproduit la quasi-totalité du spectre lumineux du soleil et est caractérisée par une température de couleur comprise entre 4 500 K et 6 500 K.
De préférence, ces lampes n'émettent pas, ou très peu, de rayons infrarouges (longueur d'onde : 780 nanomètres) et ultraviolets (longueur d'onde : 380 nanomètres) qui sont nuisibles pour la peau et la cornée.

Selon un mode de réalisation particulier, un appareil de rééducation de type banc d'optique comprend un moyen d'éclairage (6) émettant un flux lumineux dont la température de couleur est comprise entre 4 500 K et 6 500 K, de préférence entre 5 300 et 6 400 et plus préférentiellement entre 5 500 et 6 000 K.

Selon un autre mode de réalisation particulier, ledit moyen d'éclairage projectif émet une lumière spectrale composée de rayons de longueur d'onde supérieure ou égale à 380 nanomètres (violet) et inférieure ou égale à 780 nanomètres (rouge).

Un flux lumineux artificiel est également caractérisé par son indice de rendu des couleurs (IRC). Quand la température de couleur de lumière est inférieure à 5 000 K et son IRC est inférieur à 90%, la couleur jaune prédomine, cette lumière est proche de celle de la lumière artificielle, et induit une fatigue supplémentaire lors des séances de rééducation.

La luminothérapie consiste à exposer les yeux à une lumière d'intensité et de spectre lumineux proche de la lumière naturelle solaire. La perception de cette lumière par les yeux conduit le cerveau à produire des neurotransmetteurs tels que la sérotonine, qui influe sur le tonus et la capacité d'éveil et d'activité. Cette qualité de lumière influe le cerveau et le corps pour augmenter la vitalité, améliorer l'humeur et prévenir notamment les carences en vitamine D. Les indications de la luminothérapie sont nombreuses, et son efficacité pour le traitement de la dépression et des troubles du sommeil a été démontrée. La dose recommandée par les spécialistes de la luminothérapie est une exposition à une lumière d'intensité de 10 000 lux pendant 20 à 30 minutes.

Dans un appareil de rééducation selon l'invention, le moyen d'éclairage émet un flux lumineux dont la température de couleur est comprise entre 4 500 et 6 500 K, de préférence entre 5 000 K et 6 500 K, plus préférentiellement entre 5 500 et 6 500 K, les valeurs des bornes étant incluses. De préférence, le moyen d'éclairage sera choisi parmi les moyens peu énergivores.

A titre d'exemple, dans un appareil selon l'invention, une ampoule dite « lumière du jour » émettant un flux lumineux, dont la température de couleur est incluse dans une gamme de 5 400 K à 6 100 K, est adaptée ; une lampe émettant un flux lumineux dont la température de couleur est comprise entre 5 500 K et 6 500 K est également adaptée.

L'indice de rendu des couleurs (IRC) standard est de 80%. Un moyen d'éclairage d'un appareil de rééducation selon l'invention est caractérisé par un indice de rendu des couleurs supérieur ou égal à 80%, de préférence supérieur ou égal à 90%, et assurant la reproduction fidèle du rendu des couleurs, et plus préférentiellement supérieur ou égal à 91 %, supérieur ou égal à 92%, de préférence compris entre 92 et 96 %.

Ladite source lumineuse est de préférence choisie parmi les ampoules aux halogénures métalliques, ou « lampe à halogène », une lampe fluorescente, les diodes électroluminescentes (DEL en français, LED, en anglais pour Light-Emitting Diode), ou tout autre type d'ampoule adaptée. Préférentiellement, un moyen d'éclairage dans un appareil selon l'invention est choisi parmi les LED dits « lumière du jour » dont la température de couleur est incluse dans une gamme comprise entre 5 400 K et 6 100 K.

L'intensité de la lumière émise par une source lumineuse adaptée à un appareil de rééducation selon l'invention est supérieure à 1 000 Lux et inférieure ou égale à 10 000 Lux. L'utilisation d'une source d'éclairage d'une intensité lumineuse supérieure à 1 000 Lux permet notamment de resynchroniser l'horloge biologique. Plus l'intensité lumineuse augmente, plus l'effet sur l'horloge biologique et sur les structures non visuelles est important. Cette intensité est de préférence réglable à l'aide d'un potentiomètre (14), selon les besoins de la personne en rééducation.

Un appareil de rééducation neurosensorielle selon l'invention est donc adapté à la luminothérapie. Selon un mode de réalisation particulier, la présente invention a pour objet un appareil de rééducation neurosensorielle et de luminothérapie.

Selon un mode de réalisation particulier, un appareil de rééducation neurosensorielle de type banc d'optique selon l'invention comprend une poutre horizontale (1) sur laquelle sont fixés au moins lesdits dispositifs porte-filtre (5), dispositif porte-tests (7) et moyen d'éclairage projectif (6). Selon ce mode de réalisation, les distances entre les dispositifs (5), (6) et (7) et le patient ne varient pas au cours d'une séance de rééducation.

Selon un mode de réalisation plus particulier, un appareil de rééducation neurosensorielle de type banc d'optique selon l'invention comprend une poutre horizontale (1) sur laquelle sont fixés, de l'extrémité proximale à l'extrémité distale de ladite poutre : le dispositif porte-filtre (5), le moyen d'éclairage projectif (6) du dispositif porte-test, et ledit dispositif porte-test (7).

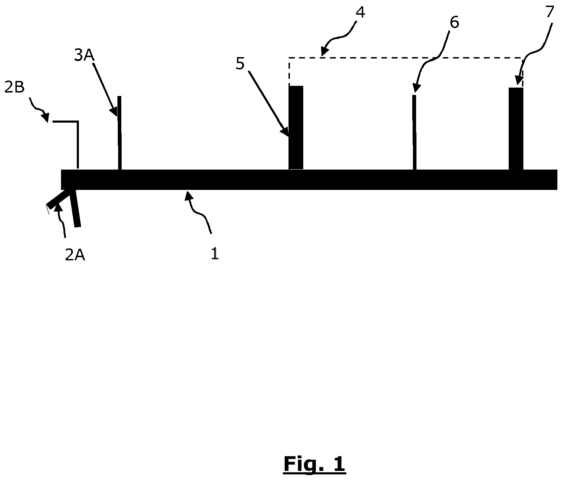

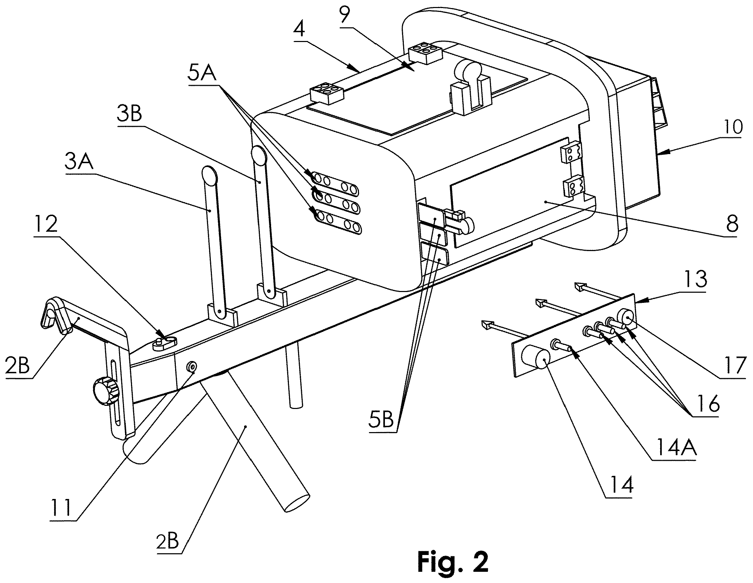

Un appareil de rééducation neurosensorielle selon l'invention (voir figures 1 et 2) comprend une enceinte opaque (4), ladite enceinte opaque intégrant le dispositif porte-filtre (5) comportant des moyens d'obturation (5A, 5B), le dispositif porte-tests (7) et le moyen d'éclairage (6) dudit dispositif porte-tests (7). La présence d'un volume clos, constitué par une enceinte opaque, ou « cartérisation » autour du porte-filtre, du moyen d'éclairage et du porte-tests canalise la lumière émise par le moyen d'éclairage dans un espace fermé. La quantité de lumière reçue par le patient est donc bien définie, contrairement au dispositif connu de l'art antérieur, qui ne comprend pas une telle enceinte. Un tel dispositif présente également l'avantage, lorsque plusieurs bancs de rééducation neurosensorielle sont utilisés dans la même pièce, d'individualiser le signal lumineux reçu par un patient sans que ce dernier soit « parasité » par la lumière d'un appareil voisin, et favorise également sa concentration. Un tel dispositif permet également de limiter la fatigue visuelle du praticien de rééducation. Une telle enceinte opaque peut être constituée en tout matériau adapté, notamment en métal, en particulier en métal peint en noir mat ou opaque, afin d'absorber la lumière et ne pas la réfléchir, ou bien en dérivés de plastique ne réfléchissant pas la lumière, tels que Polyacétate, ABS (acrylonitrile butadiène styrène).

Selon un aspect particulier de l'invention, la face proximale de l'enceinte opaque (4) intègre le dispositif porte-filtre (5) comportant des moyens d'obturation (5A, 5B) et/ou la face distale de l'enceinte opaque intègre le dispositif porte-tests (7) (voir figure 1). Selon un aspect plus particulier de l'invention, la face proximale de l'enceinte opaque (4) intègre le dispositif porte-filtre (5) comportant des moyens d'obturation (5A, 5B) et la face distale de l'enceinte opaque intègre le dispositif porte-tests (7).

Ladite enceinte opaque est munie de préférence d'au moins une ouverture latérale (8) ou au moins une ouverture supérieure (9), et de préférence une ouverture latérale (8) et une ouverture supérieure (9) (voir figure 2). Chacune de ces ouvertures peut notamment être constituée par un volet opaque ouvrant ou coulissant. Cette ou ces ouvertures permettent d'accéder aux dispositifs disposés sur le banc d'optique, et permettent au thérapeute, si nécessaire, de procéder à une correction manuelle du défaut de vision, par exemple par l'insertion d'un élément supplémentaire dans le champ de vision.

Selon un aspect particulier, dans un appareil selon l'invention, la ou les lames escamotables (3A, 3B) sont réalisées en matériau opaque, et de préférence en métal. Ladite ou lesdites lames sont destinées à supprimer certains axes lumineux. Selon un mode de réalisation particulier, un appareil selon l'invention comporte une seule lame (3A). Selon un autre mode de réalisation particulier, un appareil selon l'invention comporte deux lames (3A) et (3B). Selon un mode de réalisation plus particulier, un appareil selon l'invention comporte une (3A) ou deux lames (3A) et (3B), l'une au moins des deux lames comportant deux encoches latérales symétriques, les encoches étant de préférence de forme arrondie. Ladite au moins une lame escamotable est destinée à supprimer certains axes lumineux, la présence desdites encoches latérales permet d'adapter l'appareil à l'écartement des yeux du patient.

De préférence, dans un appareil de rééducation selon l'invention, ledit dispositif porte-filtre et/ou dispositif porte-tests se présente sous la forme de plaques porte-filtre et/ou porte-tests, tels que notamment décrits dans le brevet français N° 2 674 427. Lesdites plaques porte-filtre comportent une plaque amont percée de trous, et une plaque aval comprenant des moyens coulissants d'obturation, de préférence par réglettes montées sur des glissières, lesdites réglettes étant également percées de trous. Lesdites plaques porte-tests comportent un ou plusieurs prismes rotatifs, de préférence des prismes carrés rotatifs indexés, et des languettes coulissantes.

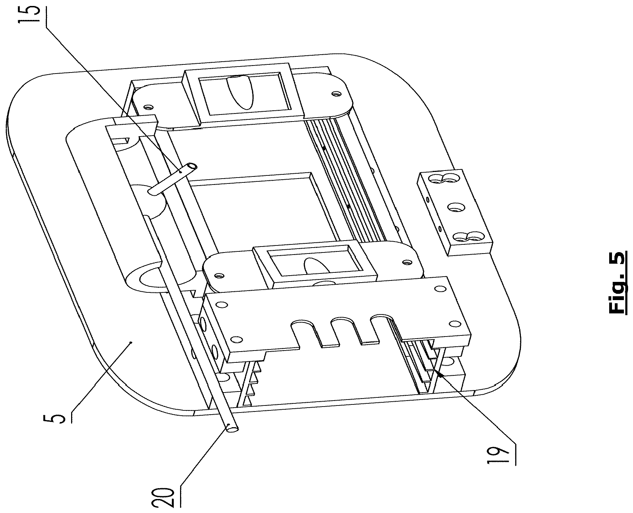

Selon un aspect particulier, ledit dispositif porte-filtre comprend un réceptacle de filtres interchangeables (19) (voir figure 5). Chaque filtre possède des cotes particulières et un emplacement prédéfini. Selon un mode de réalsiation particulier, l'appareil décrit est équipé de 4 filtres interchangeables, chacun d'eux permettant d'exploiter l'appareil pour une plage d'écartements pupillaires différents comme décrit ci-dessous, selon sa position précise par rapport au patient (c'est-à-dire par rapport à la butée de l'appui-nez). Par exemple, un filtre particulier placé à 214 millimètres du patient sera adapté pour des écartements pupillaires allant de 40 à 49 millimètres. D'autres filtres, placés entre 224 et 245 millimètres du patient, seront adaptés pour des écartements pupillaires allant de 49 à 88 millimètres et plus pour les cas exceptionnels.

Selon un autre aspect particulier, un appareil selon l'invention comprend un moyen de réglage (18) de la position de la lame escamotable (3A) ou des lames escamotables (3A) et (3B). Un tel moyen de réglage est un moyen mécanique de réglage précis, tel que notamment une queue d'aronde. La cote de positionnement de cette lame ou de ces lames escamotables sera proportionnelle au positionnement du filtre utilisé.

Ledit moyen de réglage permet de régler de façon très précise la position de la lame escamotable et donc d'adapter l'appareil de rééducation selon l'invention à des patients dont l'écartement pupillaire est différent. En effet, dans les appareils de rééducation précédemment connus, les appareils peuvent seulement être utilisés de façon optimale par des patients dont l'écartement pupillaire était de 65 millimètres plus ou moins cinq millimètres. Selon cet aspect particulier d'un appareil selon l'invention, l'appareil est utilisable de façon optimale par des patients dont l'écartement pupillaire varie de 45 à 88 millimètres.

A titre d'exemple, pour un patient dont l'écartement pupillaire est de 45 mm, on utilise un filtre caractérisé par son entre-axe et des diamètres d'orifice particuliers, ledit filtre étant placé dans le réceptacle (19) au sein du dispositif porte-filtre (5) à une distance de 214 mm des yeux du patient, la position de la lame escamotable étant réglée à une distance de 110 mm du patient.

Il apparaît clairement, d'après la présente description, que lors d'une rééducation neurosensorielle mettant en oeuvre un appareil selon l'invention, la vision du patient est binoculaire. En effet, c'est l'apprentissage de la stabilisation des images par la vision simultanée par les deux yeux du patient qui conduit progressivement à la rééducation neurosensorielle du patient.

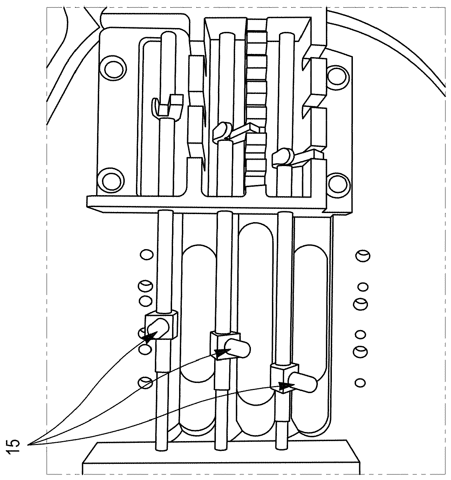

Selon un autre aspect particulier, dans un appareil de rééducation neurosensorielle selon l'invention, ledit dispositif porte-tests (7) comprend en outre au moins un dispositif escamotable comprenant un objet réflecteur ou un éclairage de couleur rouge (15) ; qui est de préférence un objet réflecteur, indicateur, témoin lumineux ou voyant de couleur rouge. Ce dispositif peut être : soit monté sur une tige coulissante qui se manipule de l'extérieur de l'enceinte close et avec un système de crantage, permettant de positionner l'indicateur sans ouvrir les volets (8) et/ou (9) ; soit monté sur un support clipsable en façade de la plaque porte test, qui impose d'ouvrir les volets (8) et/ou (9) pour leur mise en place. Dans les deux cas, chaque étage de la plaque porte-test est, de préférence, équipée d'un tel indicateur ((15) voir figure 3). Selon un aspect plus particulier, l'appareil selon l'invention comprend au moins un, et de préférence au moins deux ou trois, objet(s) réflecteur(s) (15) constitué par au moins un cylindre ou une plaque de PMMA diffusant de couleur rouge, de dimensions comprises entre 5 et 7 mm de long et 3 à 5 mm de diamètre, solidaire de chacune des réglettes montées sur glissière. Selon un autre aspect plus particulier, l'appareil selon l'invention comprend au moins un, et de préférence au moins deux, éclairage de couleur rouge, par exemple de type LED, situé sur la face antérieure ou sur la face postérieure dudit dispositif porte-test.

La commande permettant d'actionner au moins un objet réflecteur rouge ou d'allumer ou éteindre au moins un éclairage rouge est de préférence accessible au patient en cours de rééducation.

Un tel dispositif constitue un système intégré de correction d'une déformation de la vision. En effet, certains troubles nerveux se caractérisent par un trouble visuel dans lequel, après une certaine durée de fixation de plusieurs images, certaines images se superposent, s'inversent ou disparaissent. La lumière rouge, de longueur d'onde voisine de 780 nm, passe rapidement dans la paroi rétinienne, demande peu d'effort et d'énergie nerveuse. L'émission d'un voyant rouge conduit le cerveau à séparer lesdites images superposées ou à les repositionner, et participe de ce fait à la rééducation.

Selon cet aspect particulier, la présence d'au moins un élément réflecteur ou éclairage rouge permet de normaliser, recentrer et corriger le travail lors de la séance par une correction continue, et évite au praticien d'être constamment proche d'un patient lors de la rééducation. Selon les besoins particuliers du patient, l'un ou l'autre dudit objet réflecteur ou éclairage rouge est actionné, ou non.

Selon un autre aspect particulier, dans un appareil de rééducation selon l'invention, ledit dispositif porte test (7) est capable de recevoir des plaques test d'épaisseur comprise entre 5 mm et 10 mm, de préférence des plaques de 5 mm ou de 10 mm. Ceci permet notamment d'insérer dans ledit dispositif porte-test des plaques comportant des coupes de pierres ou de minéraux de différente nature.

Selon un autre aspect particulier, un appareil de rééducation selon l'invention est monté sur un pied-support à base stable, de type courant, permettant de régler la hauteur de la poutre à une distance comprise entre environ 10 cm et environ 2 m, et de préférence entre 50 cm et 2m de ladite base. Cette grande amplitude permet d'adapter l'appareil aux patients de toute taille, adultes et enfants, placés en position assise ou verticale, afin d'optimiser le confort et la stabilité du patient. De plus, ce réglage en hauteur permet d'adapter l'appareil de façon à ce que, lors de la rééducation, les pieds du patient sont en contact avec le sol, en accord avec les principes de la médecine traditionnelle chinoise.

Selon un autre aspect particulier, un appareil de rééducation selon l'invention comprend au moins un moyen de stabilisation du patient, notamment constitué par au moins un manche ou une poignée (2A), et de préférence constitué par deux poignées, tel que représenté sur la figure N°1, et/ou par un appui-nez (2B).

Selon un autre aspect particulier, un appareil de rééducation neurosensorielle selon l'invention comprend une prise destinée au branchement d'un moyen audiophonique (11). Une telle prise permet au patient de recevoir une stimulation auditive qui se combine avec la stimulation visuelle décrite précédemment. Cette stimulation auditive peut être une stimulation musicale, dont les caractéristiques sont choisies par le thérapeute selon les besoin thérapeutiques du patient, qui bénéficie ainsi de la combinaison de la musicothérapie et de la rééducation visuelle.

Selon un autre aspect particulier, un dispositif selon l'invention comprend un boitier ou tableau de commande situé à l'arrière de l'enceinte opaque (4) par rapport au patient en cours de rééducation. La localisation de ces moyens de commande évite toute manipulation non souhaitée.

Selon un aspect encore plus particulier, la présente invention a pour objet un appareil de rééducation de type banc d'optique comprenant une poutre horizontale (1), éventuellement graduée, sur laquelle sont disposés, de l'extrémité proximale à l'extrémité distale de la poutre : au moins un moyen de stabilisation des mains et du corps (2A) adapté à une personne située à l'extrémité proximale de la poutre, un appui nez (2B), au moins une lame escamotable par rotation (3A, 3B), un dispositif porte-filtre (5) comportant des moyens d'obturation par réglettes montées sur glissières (5A, 5B), un moyen d'éclairage projectif (6) d'un dispositif porte-tests (7), un dispositif porte-tests (7) comprenant des prismes dont les faces portent des signes et/ou des couleurs, et de préférence des prismes carrés rotatifs indexés, ladite poutre (1) étant montée sur un pied-support permettant le réglage en hauteur de la poutre, ledit banc d'optique étant caractérisé en ce que ledit moyen d'éclairage (6) émet un flux lumineux dont la température de couleur est comprise entre 4 500 K et 6 500 K et en ce que ledit moyen d'éclairage (6), le dispositif porte-filtre (5A, 5B) et le dispositif porte-tests (7) sont intégrés dans une enceinte opaque (4) munie d'au moins une ouverture (8)(9).

Selon un autre mode de réalisation particulier, l'invention a pour objet un appareil de rééducation comprenant une poutre horizontale (1) sur laquelle sont fixés, de l'extrémité proximale à l'extrémité distale de ladite poutre : un moyen de stabilisation (2A) adapté à une personne située à l'extrémité proximale de la poutre, au moins une lame (3A) escamotable par rotation, un dispositif porte-filtre (5) comportant des moyens d'obturation (5A, 5B), un moyen d'éclairage projectif (6) d'un dispositif porte-tests (7) et un dispositif porte-tests (7) comprenant des prismes dont les faces portent des signes et/ou des couleurs, ladite poutre étant montée sur un pied-support permettant le réglage en hauteur de la poutre, ledit banc d'optique étant caractérisé en ce que ::
- ledit moyen d'éclairage (6) émet un flux lumineux dont la température de couleur est comprise entre 4 500 K et 6 500 K, de préférence entre 5 300 et 6 400, plus préférentiellement entre 5 500 et 6 000 K, et
- ledit moyen d'éclairage (6), le dispositif porte-filtre (5) et le dispositif porte-tests (7) sont intégrés dans ladite enceinte opaque (4).

Selon d'autres aspects plus particuliers, un banc de rééducation neurosensorielle selon l'invention comprend également :
- au moins un dispositif de minuterie commandant ledit moyen d'éclairage, les durées de minuterie, correspondant à la durée de la rééducation, pouvant être de 5, 10, 20 ou 30 mn, ce dispositif permettant de maîtriser la durée de la stabilisation de l'image fixée par le patient, ce dispositif peut être couplé à une horloge à alarme programmable et affichage du temps écoulé ou du temps restant de rééducation, il peut également inclure l'émission d'un signal sonore ou l'allumage d'un voyant à la l'issue du temps programmé, et/ou
- un potentiomètre permettant de faire varier l'intensité de l'éclairage dans l'enceinte opaque, selon les conditions de rééducation souhaitées, ce variateur d'intensité permet un réglage très fin du rendu lumineux variant de 0 à 250 lumens.
- au moins une lame escamotable rectiligne (3A), éventuellement munie de deux encoches latérales symétriques de forme arrondie : ladite ou lesdites lames escamotables sont destinées à supprimer certains axes lumineux, la présence desdites encoches latérales permet d'adapter l'appareil à l'écartement des yeux du patient.
- un dispositif porte-pierres, destiné à intégrer dans le champ de vison du patient une ou des pierres naturelles translucides, choisies selon la longueur d'onde de la lumière traversant chacune des pierres. Ledit dispositif porte pierres est de préférence inséré dans le dispositif porte-tests.

Selon la distance comprise entre l'oeil du patient et la plaque porte-tests, un appareil de rééducation selon l'invention correspond à l'un des huit bancs optiques suivants :
- « couleurs spectrales » ou préparatoire
- « vision au loin » horizontal fixe
- « vision au loin » périmétroscopique
- « vision intermédiaire fixe »
- « vision intermédiaire périmétroscopique »
- « vision prossima fixe »
- « haute-tension fixe »
- « haute-tension périmétroscopique ».

Ces différents appareils de rééducation selon l'invention permettent :
- une rééducation de la vision de loin, ou repos, lorsque la distance entre l'oeil et le dispositif porte-tests est de 120 cm,
- une rééducation de la vision intermédiaire, ou « effort moyen », lorsque la distance entre l'oeil et le dispositif porte-tests est de 60 cm
- une rééducation de la vision de près, ou « grand effort », lorsque la distance entre l'oeil et le dispositif porte-tests est de 30 cm
- une rééducation de la vision de près, ou « haute tension », lorsque la distance entre l'oeil et le dispositif porte-tests est de 30 cm.

L'exemple qui suit illustre l'invention de façon plus détaillée, sans toutefois en limiter la portée.

### EXEMPLE 1

Un appareil de rééducation neurosensorielle de type banc d'optique selon l'invention (voir figures 1 et 2) et correspondant à une application de type « Effort moyen » comprend une poutre horizontale (1), fixée sur un pied-support non visible sur la figure, sur laquelle sont fixés, de son extrémité proximale à son extrémité distale :
- deux poignées de stabilisation (2A),
- un appui nez réglable en hauteur (2B),
- une première lame escamotable rectiligne de 19 mm (3A), située à 153 mm du dispositif porte test,
- une seconde lame escamotable rectiligne de 10,5 mm (3B), située à 93 mm du dispositif porte test,
- une enceinte opaque (4) dont la surface proximale comprend un dispositif porte-filtre (5) comprenant des moyens d'obturation (5A, 5B), munie d'un volet latéral de dimensions 90 x 130 mm (8) et d'un volet supérieur de dimensions 160 x 170 mm (9), les deux volets ouvrants étant maintenus par des crochets, assurant ainsi la fermeture de l'enceinte de façon hermétique à la lumière,
- un moyen d'éclairage constitué par une ampoule « lumière du jour » émettant un flux lumineux d'environ 6 000 K (6),
- une plaque porte-tests intégrée dans la surface distale de l'enceinte opaque (7),
- Un boitier (10) de dimensions 220 x 130 x 100 mm comportant des rayonnages, et destiné au rangement des différentes plaques,
- Une prise pour un casque audio (11),
- Un voyant de fin de cycle de rééducation (12),
- Un panneau de commande (13) comprenant un potentiomètre (14) réglant l'intensité de lumière diffusée par le moyen d'éclairage, un dispositif d'interruption de l'éclairage, des dispositifs de minuterie de 10, 20 ou 30 mn (16), un fusible (17),
- Une alimentation électrique (non visible sur les figures).

Lors de la séance de rééducation, le patient observe simultanément avec les deux yeux les signes ou les images qui lui sont présenté(e)s.

### EXEMPLE 2

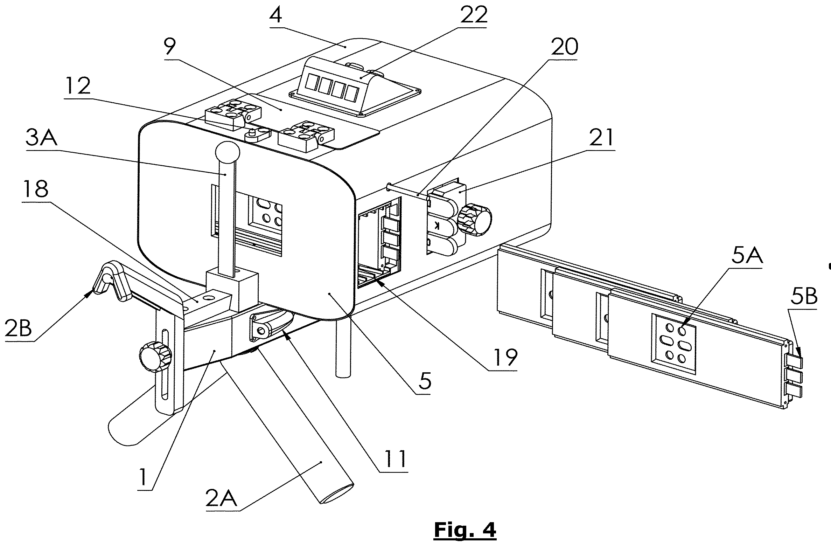

Un mode de réalisation particulier d'un appareil de rééducation neurosensorielle selon l'invention est représenté sur les figures 4 et 5. Cet appareil comprend en particulier :
- un moyen de réglage mécanique de type queue d'aronde (18) permettant le réglage de la lame escamotable (3A).
- un dispositif porte-filtre (5) comprenant des moyens d'obturation (filtres) (5A) et (5B) interchangeables, et un réceptacle de filtres interchangeables (19),
- un dispositif escamotable comprenant un éclairage de couleur rouge (15) commandé par une tige de commande (20),
- un dispositif porte-tests (7) susceptible de recevoir des plaques de 5 ou 10 mm d'épaisseur, et comprenant une cale (21) permettant d'adaptation à l'épaisseur des plaques,
- une horloge digitale programmable (22).

Dans la figure 4, le dispositif porte-tests (7) et le moyen d'éclairage projectif (6) dudit dispositif porte-test sont inclus au sein de l'enceinte opaque et ne sont donc pas visibles.

La figure 5 montre une représentation schématique de l'arrière d'un dispositif porte-filtre (5), comprenant un moyen d'éclairage projectif (6) maintenu par un support, un éclairage de couleur rouge (15) commandé par une tige de commande (20), et un réceptacle de filtres interchangeables (19).

Selon un mode de réalisation particulier d'un appareil selon l'invention, le dispositif porte-test est susceptible de recevoir des plaques porte-tests dans lesquelles sont insérées des éléments translucides, tels que des pierres Dans ce cas particulier, lesdits éléments translucides sont éclairés par l'arrière par un éclairage de type « lumière du jour » fixé à l'arrière dudit dispositif porte-test.

## Revendications

1. Appareil de rééducation de type banc d'optique comprenant une poutre horizontale (1) comprenant :
- un dispositif porte-filtre (5) comportant des moyens d'obturation (5A, 5B),
- un dispositif porte-tests (7), comprenant des moyens de support de signes et/ou de couleurs,
- un moyen d'éclairage projectif (6) dudit dispositif porte-tests (7),
- une enceinte opaque (4),
ledit appareil de rééducation étant **caractérisé en ce que** :
- ledit moyen d'éclairage (6) émet un flux lumineux dont la température de couleur est comprise entre 4 500 K et 6 500 K et
- ledit moyen d'éclairage (6), le dispositif porte-filtre (5) et le dispositif porte-tests (7) sont intégrés dans ladite enceinte opaque (4).

2. Appareil de rééducation selon la revendication 1, **caractérisé en ce que** l'enceinte opaque (4) est munie d'au moins une ouverture latérale ou au moins une ouverture supérieure.

3. Appareil de rééducation selon l'une quelconque des revendications 1 ou 2, **caractérisé en ce qu'**il comprend en outre au moins un moyen de stabilisation, notamment au moins un manche ou au moins une poignée (2A) et/ou un appui-nez (2B).

4. Appareil de rééducation selon l'une quelconque des revendications 1 à 3, **caractérisé en ce que** ledit dispositif porte-tests comprend en outre au moins un dispositif escamotable comprenant un objet réflecteur ou un éclairage de couleur rouge (15).

5. Appareil de rééducation selon l'une quelconque des revendications 1 à 4, **caractérisé en ce qu'**il comprend en outre une prise destinée au branchement d'un moyen audiophonique (11).

6. Appareil de rééducation selon l'une quelconque des revendications 1 à 5, **caractérisé en ce qu'**il comprend en outre une minuterie (16)

7. Appareil de rééducation selon l'une quelconque des revendications 1 à 6, **caractérisé en ce qu'**il comprend en outre un potentiomètre d'intensité (14) dudit moyen d'éclairage (6).

8. Appareil de rééducation selon l'une quelconque des revendications 1 à 7, **caractérisé en ce qu'**il comprend en outre au moins une lame escamotable (3A), ladite lame étant munie de deux encoches latérales, de préférence de forme arrondie.

9. Appareil de rééducation selon l'une quelconque des revendications 1 à 8, **caractérisé en ce qu'**il comprend deux lames escamotables (3A) et (3B).

10. Appareil de rééducation selon l'une quelconque des revendications 1 à 9, comprenant une poutre horizontale (1) sur laquelle sont disposés, de l'extrémité proximale à l'extrémité distale de ladite poutre : un moyen de stabilisation (2A) adapté à une personne située à l'extrémité proximale de la poutre, au moins une lame (3A) escamotable par rotation, un dispositif porte-filtre (5) comportant des moyens d'obturation par réglettes montées sur glissières (5A, 5B), un moyen d'éclairage projectif (6) d'un dispositif porte-tests (7) et un dispositif porte-tests (7) comprenant des prismes dont les faces portent des signes et/ou des couleurs, ladite poutre étant montée sur un pied-support permettant le réglage en hauteur de la poutre, ledit banc d'optique étant **caractérisé en ce que** ::
- ledit moyen d'éclairage (6) émet un flux lumineux dont la température de couleur est comprise entre 4 500 K et 6 500 K et
- ledit moyen d'éclairage (6), le dispositif porte-filtre (5) et le dispositif porte-tests (7) sont intégrés dans ladite enceinte opaque (4).
